(19) Europäisches Patentamt
European Patent Office
Office européen des brevets

(11) **EP 4 078 597 B1**

(12) **EUROPEAN PATENT SPECIFICATION**

(45) Date of publication and mention
of the grant of the patent:
**17.09.2025 Bulletin 2025/38**

(21) Application number: **20718583.6**

(22) Date of filing: **01.04.2020**

(51) International Patent Classification (IPC):
**G16B 40/20** (2019.01)    **G16B 50/10** (2019.01)
**G16H 50/20** (2018.01)    **G16H 50/70** (2018.01)

(52) Cooperative Patent Classification (CPC):
**G16B 50/10; G16B 40/20; G16H 50/20;
G16H 50/70**

(86) International application number:
**PCT/EP2020/059317**

(87) International publication number:
**WO 2021/197602 (07.10.2021 Gazette 2021/40)**

(54) **METHOD AND SYSTEM FOR LEARNING NOVEL RELATIONSHIPS AMONG VARIOUS BIOLOGICAL ENTITIES**

VERFAHREN UND SYSTEM ZUM ERLERNEN NEUER BEZIEHUNGEN ZWISCHEN VERSCHIEDENEN BIOLOGISCHEN EINHEITEN

PROCÉDÉ ET SYSTÈME D'APPRENTISSAGE DE NOUVELLES RELATIONS ENTRE DIVERSES ENTITÉS BIOLOGIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**26.10.2022 Bulletin 2022/43**

(73) Proprietor: NEC Corporation
**Minato-ku
Tokyo 108-8001 (JP)**

(72) Inventors:
• **SZTYLER, Timo
69434 Hirschhorn (DE)**
• **MALONE, Brandon
69221 Dossenheim (DE)**

(74) Representative: **Ullrich & Naumann PartG mbB
Schneidmühlstrasse 21
69115 Heidelberg (DE)**

(56) References cited:
**US-A1- 2019 370 616**

• **PENG B ET AL: "CNN-based Dual-Chain Models for Knowledge Graph Learning", ARXIV.ORG, CORNELL UNIVERSITY LIBRARY, 201 OLIN LIBRARY CORNELL UNIVERSITY ITHACA, NY 14853, 16 November 2019 (2019-11-16), XP081533824**
• **HENG CHUNG M W ET AL: "Clinical Knowledge Graph Embedding Representation Bridging the Gap between Electronic Health Records and Prediction Models", 2019 18TH IEEE INTERNATIONAL CONFERENCE ON MACHINE LEARNING AND APPLICATIONS (ICMLA), IEEE, 16 December 2019 (2019-12-16), pages 1448 - 1453, XP033719706, DOI: 10.1109/ ICMLA.2019.00237**
• **MORALES C ET AL: "MateTee: A Semantic Similarity Metric Based on Translation Embeddings for Knowledge Graphs", 1 June 2017, BIG DATA ANALYTICS IN THE SOCIAL AND UBIQUITOUS CONTEXT : 5TH INTERNATIONAL WORKSHOP ON MODELING SOCIAL MEDIA, MSM 2014, 5TH INTERNATIONAL WORKSHOP ON MINING UBIQUITOUS AND SOCIAL ENVIRONMENTS, MUSE 2014 AND FIRST INTERNATIONAL WORKSHOP ON MACHINE LE, ISBN: 978-3-642-17318-9, XP047415848**

- **XIE R ET AL:** "Knowledge graph representation learning method and system in combination with entity description", 1 January 2016 (2016-01-01), XP055754141, Retrieved from the Internet <URL:https://hcsi.cs.tsinghua.edu.cn/Paper/Paper16/AAAI16-RuobingXie.pdf> [retrieved on 20201125]
- **XIAO H ET AL:** "SSP: Semantic Space Projection for Knowledge Graph Embedding with Text Descriptions", ARXIV.ORG, CORNELL UNIVERSITY LIBRARY, 201 OLIN LIBRARY CORNELL UNIVERSITY ITHACA, NY 14853, 17 April 2016 (2016-04-17), XP080695970
- **GARCIA-DURAN A ET AL:** "Learning Graph Representations with Embedding Propagation", ARXIV.ORG, CORNELL UNIVERSITY LIBRARY, 201 OLIN LIBRARY CORNELL UNIVERSITY ITHACA, NY 14853, 9 October 2017 (2017-10-09), XP080827188
- **ZHOU W ET AL:** "Knowledge Graph Embedding With Interactive Guidance From Entity Descriptions", IEEE ACCESS, vol. 7, 29 October 2019 (2019-10-29), pages 156686 - 156693, XP011757018, DOI: 10.1109/ACCESS.2019.2950015
- **WANG K ET AL:** "Knowledge Graph Embedding with Entity Neighbors and Deep Memory Network", ARXIV.ORG, CORNELL UNIVERSITY LIBRARY, 201 OLIN LIBRARY CORNELL UNIVERSITY ITHACA, NY 14853, 11 August 2018 (2018-08-11), XP081255965
- **BORDES A ET AL:** "Translating Embeddings for Modeling Multi-relational Data", ADVANCES IN NEURAL INFORMATION PROCESSING SYSTEMS 26 (NIPS 2013), 5 December 2013 (2013-12-05), Lake Tahoe, Stateline, NV, USA, XP055298868

**Description**

The present invention relates to a computer-implemented method as well as to a computer system for learning novel relationships among various entities, in particular biological entities such as chemicals, proteins, and diseases.

Biological processes within the human body are the result of complex interactions among various molecules, such as DNA, RNA, protein, metabolites, enzymes, and more. Disease phenotypes result from the disruption of these processes; likewise, effective treatments aim to restore these processes to their normal, healthy state. Thus, it is imperative to accurately and thoroughly characterize the relationships among the molecules, diseases, and other entities involved in these processes.

Traditional approaches for characterizing these relationships entail expensive and time-consuming biological wet lab experiments. Due to the cost of these experiments, existing datasets are highly incomplete. Thus, while this experimental approach remains the gold standard for determining their truth, it is important to devise more cost-effective methods to characterize these relationships.

Increasing evidence demonstrates that artificial intelligence (AI) methods are also very effective for identifying novel relationships. Of course, high-confidence relationships predicted by the AI methods can (and should) still be validated with traditional wet lab approaches.

To address this problem, network-based approaches have been proposed already in prior art, in which each entity is represented as a node in a graph and each relationship is represented as an edge between the respective entities. Different relationship types are represented with different edge types. Such network-based approaches can be grouped into three categories: neighborhood-based, diffusion-based, and representation-based. These rely on different topological properties of the network, such as motifs, communities, hubs, clusters, node centrality, shortest paths, and others to predict new edges. Further, some methods also account for biological features of the nodes.

For instance, considering the representation-based approaches, which are the most similar to the present invention, these approaches use neural networks to learn embeddings, or real-valued numeric vectors, such that entities which are close in the graph and have similar relationships also have similar embeddings. A machine learning model is then trained to predict novel relationships based on the embeddings.

Peng et al. (2019) discloses a convolutional neural network-based model which directly captures interactions among relations and entities over their embeddings.

US2019/370616A1 discloses methods for predicting an association between input data in a first modality and data in a second modality using a statistical model comprising a plurality of encoders and decoders, each of which is trained to process data for one of the plurality of modalities, and a joint-modality representation coupling the plurality of encoders and decoders.

The known solutions mentioned above, however, prove to be disadvantageous since all existing approaches are limited by the network structure.

It is therefore an object of the present invention to improve and further develop a method and system of the initially described type for learning novel relationships among various entities in such a way that the view of the knowledge graph is improved.

The invention is defined in the appended claims.

In an embodiment, the present invention provides a computer-implemented method of learning novel relationships among biological entities including chemicals, genes and/or proteins, and diseases, the method comprising:

establishing a knowledge graph wherein each of the entities is represented as a node and each relationship between the entities is represented as an edge between the respective nodes,
annotating entities in the knowledge graph with objects of one or more data modalities,
training a neural network system with the knowledge graph, wherein the neural network system treats the knowledge graph and the objects of a respective one of the data modalities in a unified manner by jointly learning embeddings of the nodes from the knowledge graph and embeddings of the objects of the respective one of the data modalities, wherein the neural network system is configured to minimize for a particular positive sample a loss function that calculates the Euclidean distance between a distance between the embeddings of the nodes from the knowledge graph for the positive sample and a center of the respective negative samples and a distance between the embeddings from the object of a particular of the data modalities for the positive sample and a center of the negative samples;
using the learned embeddings for identifying novel relationships among the entities, including selecting a particular disease and using the neural network system to predict for the selected disease relationships of the form 'gene/protein associated with disease' or of the form 'chemical treats disease';
ranking the predicted genes/proteins or chemicals according to a likelihood of the respective one of the predicted genes/proteins or chemicals to be associated with the selected disease or to treat the selected disease; and
selecting a predefined number of the top-ranked genes/proteins as candidates for a knockdown experiment or of the top-ranked chemicals as candidates for personalized drug development.

The invention also provides a corresponding computer systems for learning novel relationships among biological entities including chemicals, genes and/or proteins, and diseases.

Moreover, the invention also provides a corresponding non-transitory computer-readable medium having instructions thereon, which, upon execution by one or more processors, alone or in combination, and using memory, provides for execution of the above method.

According to the invention it has first been recognized that the above objective can be accomplished by a method that learns consistent representations of multiple data modalities, in particular biological entities, such as proteins and diseases, based on a knowledge graph, KG, and any additional data modality, like structured annotations and free text describing the entities. These representations may then be used to identify novel relationships, e.g., proteins that are associated with diseases. The novel relationships could be used to prioritize protein targets for new drugs. In contrast to prior art approaches, embodiments of the invention explicitly incorporate the consistency of the representations into the training process. In particular, embodiments of the invention consider the KG structure and documents about the entities in a unified manner. More specifically, uniting the embeddings and learning to predict true relationships is performed in a single step, i.e. the embeddings and the prediction model are learned jointly. This ensures that the embeddings are optimized for prediction.

Embodiments of the present invention relate to a method and system for learning novel relationships among the various biological entities, like proteins, drugs, and diseases, adopting a network-based approach in which each entity is represented as a node in a graph and each relationship is represented as an edge between the respective entities, wherein different relationship types are represented with different edge types. In the context of the present disclosure such network is referred to as a knowledge graph (KG). Learning novel relationships is then equivalent to predicting "missing" edges from the KG.

An obvious approach to combine the KG structure and documents (which is present in the literature) is to train independent ML models on the KG and documents and then combine them in some post hoc manner. In the context of neural network embeddings, this corresponds to learning one embedding from the KG structure and one from the documents for each entity; however, even when trained jointly and end-to-end (as done in previous work), these two embeddings are still likely to be completely different for each entity. That is, the embeddings are not unified, but, rather, completely independent.

To address this issue, embodiments of the invention relate to a link prediction system that implements a neural network learning strategy which combines the KG structure with the documents in a unified framework. In contrast to the "obvious" approach, both embeddings are forced to be similar. The main advantage compared to the obvious approach is that this "pulls together" the embeddings of entities which are distant in the KG but have similar documents. Likewise, it "pushes apart" embeddings of genes that are close in the KG but have very different documents. Thus, novel relationships are predicted using a unified view of the KG and documents. Consequently, these embodiments come along with the advantage that the neural network system learns embeddings of entities which unite the KG structure and additional information from the documents. In particular, the neural network system can learn similarities of documents based on the context of their associated entities, regardless of the syntactic or semantic overlap of their vocabulary.

In an embodiment, the present invention provides a method for identifying new candidate targets for drugs by predicting genes associated with diseases.

In another embodiment, the present invention provides a method for drug repurposing, in which an existing drug is used to treat a new disease, by predicting 'drug -- treats -- disease' edges. This is particularly relevant for immune checkpoint inhibitors (ICIs), which have been shown to be very effective for some disease but have severe side effects for others. ICIs are commonly used in combination with neoantigen-based personalized cancer vaccines.

There are several ways how to design and further develop the teaching of the present invention in an advantageous way. To this end it is to be referred to the dependent claims on the one hand and to the following explanation of preferred embodiments of the invention by way of example, illustrated by the figure on the other hand. In connection with the explanation of the preferred embodiments of the invention by the aid of the figure, generally preferred embodiments and further developments of the teaching will be explained. In the drawing

Fig. 1    is a conceptual overview of a framework for learning novel relationships among various biological entities in accordance with an embodiment of the present invention, and

Fig. 2    is a schematic overview of a neural network structure used in a method for learning novel relationships among various biological entities in accordance with an embodiment of the present invention.

In the following description of preferred embodiments of the present invention, the following notation will be used:

| Parameter | Description |
|---|---|
| $G$ | Knowledge Graph |
| $V$ | Set of nodes |
| $E$ | Set of edges |
| $e^t$ | Edge Types |
| $\boldsymbol{D}$ | Set of documents |
| $d$ | Element of $\boldsymbol{D}$ (a document) |
| $v$ | Element of $\mathbf{V}$ (a node) |
| $G_B$ | Knowledge Graph with Documents |
| $h$ | "Head" which occurs in $\mathbf{V}$ |
| $t$ | "Tail" which occurs in $\mathbf{V}$ |
| $r$ | "Relation" which occurs in $\mathbf{E}$ |
| $V_p$ | Set of all protein nodes |
| $V_p^+$ | Set of proteins that are associated with a certain disease |
| $V_p^-$ | All proteins which are not part of $V_p^+$ |
| $\boldsymbol{D}_p^+$ | Set of documents which describe the proteins in $V_p^+$ |
| $\boldsymbol{D}_p^-$ | Set of documents which describe the proteins in $V_p^-$ |
| $v_j$ | Element in $V_p^+$ |
| $d_j$ | Element in $\boldsymbol{D}_p^+$ |
| $x_s$ | Training sample which consists of nodes and documents (positive and negative samples) |
| $V_s^-$ | A subset of $V_p^-$ |
| $\boldsymbol{D}_s^-$ | A subset of $\boldsymbol{D}_p^-$ |
| $Emb_U$ | Embedding function for positive elements |
| $Emb_U^{NEG}$ | Embedding function of negative elements |
| $Emb^R$ | Embedding function of the relation types |
| $x_i$ | Placeholder for $v_j$ or $d_j$ |
| $x_j$ | Placeholder for $V_s^-$ or $\boldsymbol{D}_s^-$ |
| $U$ | Placeholder for $V$ or $\boldsymbol{D}$ |
| $r_k$ | A particular relation type |

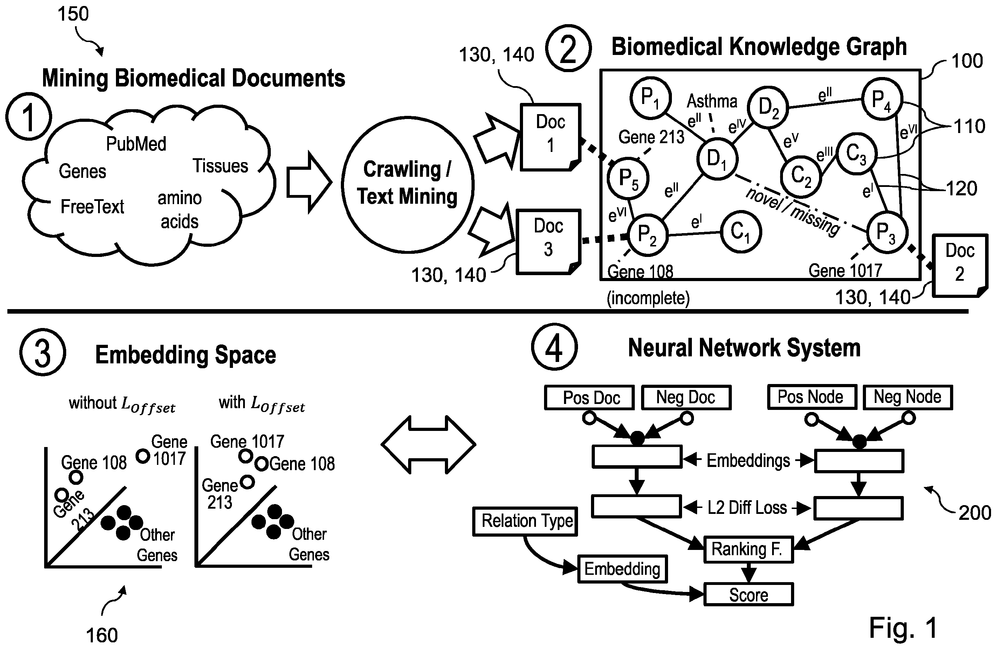

Fig. 1 depicts an overall conceptual framework of a system for learning novel relationships among various biological entities in accordance with an embodiment of the invention. According to the embodiment illustrated in Fig. 1, the framework comprises different aspects and components, including a mining biomedical documents component 150 (shown in Part 1), a biomedical knowledge graph, KG, 100 (shown in Part 2), an embedding space 160 (shown in Part 3), and a neural network system 200 (shown in Part 4). The concrete embodiment is situated in a drug development process. However, as will be appreciated by those skilled in the art, in other embodiments the framework can be related likewise to other applications.

## Biomedical Knowledge Graph

According to an embodiment of the invention, the biomedical knowledge graph 100, denoted herein $G = (V, E)$, may consist of a set of nodes 110, denoted $V$, and a set of edges 120, denoted $E$. Each node $V$ in the biomedical knowledge graph 100 corresponds to one entity, such as a chemical, a protein, or a disease. The edges $E$ characterize the known relationships between these entities.

In an embodiment, the construction of the knowledge graph $G$ may be performed by manually crafting the graph by considering various structured data sources. Specifically, according to an embodiment entities and their relationships may be extracted from various sources as follows:

| | |
|---|---|
| - from DisGeNET: | "disease -- associated_with -- protein" relationships |
| - from SIDER: | "chemical -- treats -- disease" relationships |
| - from STITCH: | "chemical -- reacts_with -- chemical relationships |
| - from STITCH: | "chemical -- interacts_with -- protein" relationships |
| - from STRING | "protein -- interacts_with -- protein" relationships |

According to an embodiment, a published approach may be followed to additionally create "disease -- similar_to -- disease" relationships.

Further, as some data sources provide confidence values for the relationships, these may be taken into account to filter relationships with a low reliability since those are already predictions.

In total, then, according to the described embodiment, the knowledge graph 100 considers three types of entities: diseases, genes (proteins), chemicals (drugs). Further, it includes six relationship (edge) types. According to the notation used herein, $e^t$ refers to edge type $t$.

As will be appreciated by those skilled in the art, other entities, data sources, and relationship extraction strategies than the ones explicitly mentioned above could also be used to construct the KG 100.

In the following, the facts which are encoded by the graph in $G$ are represented as a set of triples of the form (h, r, t), where $h \in V$ and $t \in V$ are the head and tail entities and $r \in E$ is a relation type.

As an example, Figure 1, Part 2 illustrates an excerpt of a KG 100 according to an embodiment of the invention. It pictures, for instance, that the disease *"Asthma"* ($D_1$) is associated with "Gene 108" ($P_2$) and "Gene 142" ($P_1$). Hence, written as a triple of the above form, a relation present in the KG 100 could be expressed as ($D_1$, 'is associated with', $P_2$). From a practical point of view, it is expensive to identify novel relations and most of them are still unknown.

## Mining Biomedical Documents

According to embodiments of the invention, the entities in the KG 100 (which can be considered as objects of a first data modality) are annotated with objects 130 of one or more further data modalities. In the described embodiment, the entities in the KG 100 are annotated with objects 130 of one further data modality, namely biomedical documents. The biomedical documents describe the related entities in the KG 100 in more detail. For instance, proteins may be annotated with their functions or text from academic papers in which they are described. Hereinafter, this additional information is simply referred to as a document 140 for the respective entity.

According to embodiments, the documents 140 may be retrieved by manually or automatically crawling various data sources, including unstructured sources, like academic papers from PubMed, or structured data sources, such as ontologies like the Gene Ontology. Possible details that are described by the documents 140 are, for instance, location (e.g., in which tissue is a particular protein active), biochemical properties (e.g., the hydrophobicity or molecular weight of a drug), functional behavior (e.g., a description of the phenotypes of individuals with a disease), or higher-level description (such as the outcome of a clinical trial involving a particular drug and disease).

According to embodiments, all documents 140 may be prepared for further use based on a common vocabulary. As an example, word segmentation-based or frequency-based approaches could be used. Further, standard preprocessing steps like tokenization, lemmatization, and stemming could be used. Fig. 1, Part 1 illustrates schematically an embodiment of a mining biomedical documents component 150 for creating the documents 140 from raw data sources.

Additionally, each document 140 is associated with at least one of the entities from the KG 100. A document 140 can be associated with more than one entity, and each entity can be associated with more than one document 140. Many approaches can be used to assign documents 140 to entities. For structured data sources, entity identifiers are typically given; for unstructured sources, natural language processing approaches like named entity recognition could be used to extract entity identifiers from the text. The present invention is not limited with regard to the exact approach used for creating the biomedical documents 140 from the raw data sources and matching them to entities.

According to embodiments, the output of the mining biomedical documents component 150 may be a set of biomedical documents **D**, where each $d \in \textbf{D}$ is a multiset of tokens from the vocabulary. Further, $\forall\, d \in \textbf{D}$, it holds that $\exists\, (v, d) \in (V, \textbf{D})$ where $(V, \textbf{D})$ is a set of pairs. This is denoted as $G_B = ((V, E), (V, \textbf{D}))$. $G_B$ will be used as input for the neural network component 200.

According to embodiments of the invention, the following exemplary information may be extracted from various public sources in order to create the documents 140:

- Gene Ontology: protein functions and locations in the cell (structured annotations)
- Human Phenotype Ontology: phenotypes associated with diseases (structured annotations)
- SIDER, OFFSIDES: chemical (drug) side effects (structured annotations)
- MyGene.info: protein descriptions (free text)
- DrugBank: drug descriptions (free text)
- DisGeNET: disease mechanisms and associated mutations (free text)

Fig. 1, Part 2 illustrates an embodiment of a connection between documents 140 and proteins $P_i$. In general, all entities may have associ ated documents 140, and documents 140 may be associated with multiple entities.

All current approaches known in prior art are limited by the network structure. In particular, they do not consider the structure of the KG 100 and documents 140 about the entities in a unified manner. Embodiments of the invention address this shortcoming with a machine learning-based approach in which the knowledge graph, KG, structure 100 on the one hand and documents 140 on the other hand are combined in a unified manner, as will be described in detail below.

**Neural Network System**

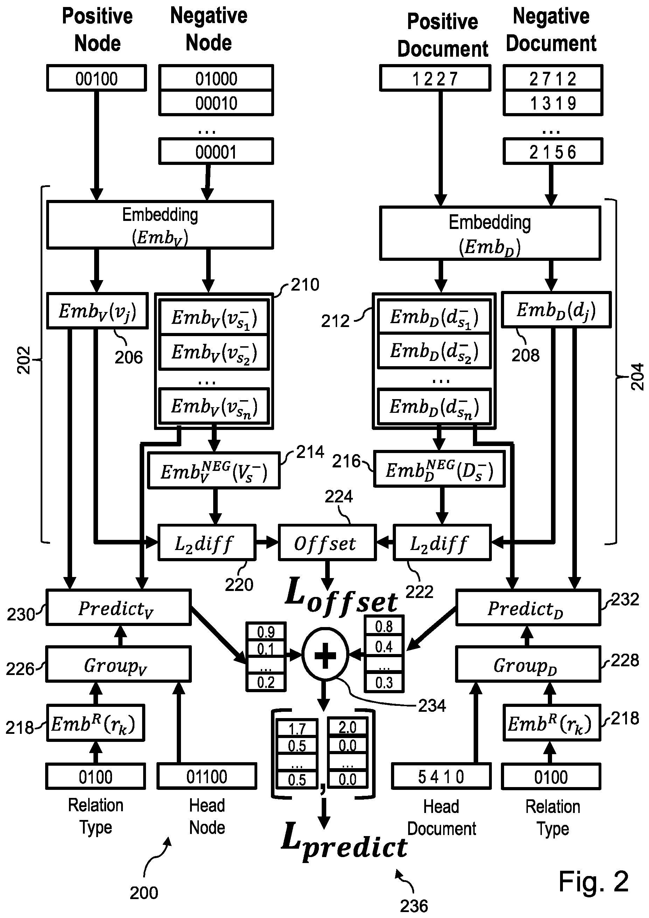

An embodiment of a neural network system 200, schematically illustrated in Fig. 1, Part 4, is depicted in Fig. 2 in greater detail. The neural network system 200 maintains the machine learning models that are responsible for predicting novel relations based on the provided biomedical knowledge graph $G_B$, as described above, and the related set of biomedical documents **D**. Specifically, Fig. 2 graphically presents the neural network structure described hereinafter in detail.

*Training*

For illustration, the following description exemplarily refers to *"proteins"* and *"diseases"* as possible head and tail entities where the corresponding relation is *"associated_with"* ($e^{d\_aw\_p}$). It should be noted that the remaining combinations of head and tail entities with corresponding relations are trained in a corresponding way.

For training a machine learning model, a particular disease (e.g. "Cardiomyopathy") and a particular relation $r_k$ (e.g. "associated_with") are kept constant as the head $h$ and relation type $r$, respectively, and the proteins are grouped into $V_p^+$ and $V_p^-$ where $\forall v_p^+ \in V_p^+$ it holds that a triple in the form of (*Cardiomyopathy, associated_with,* $v_p^+$) exists in KG. It holds that $V_p^+ \cap V_p^- = \emptyset$, $V_p^+ \cup V_p^- \subseteq V_p$ and $V_p \subseteq V$.

Analogously, $\textbf{D}_p^+$ and $\textbf{D}_p^-$ describe the corresponding biomedical documents of the elements in $V_p^+$ and $V_p^-$. That is, every protein in $V_p^+$ is a correct tail for the triple, while every protein in $V_p^-$ is an incorrect tail.

As indicated in Fig. 2, the machine learning model is conceptually trained with a single sample at a time. That is, $v_j \in V_p^+$ and the corresponding document $d_j \in \textbf{D}_p^+$ are chosen and, as a first step, this sample is embedded, where the embedding of the nodes V and the documents **D** happens independently, as shown at 202 for the nodes V and 204 for the documents **D**.

According to an embodiment, as shown at 206 for the nodes V and at 208 for the documents **D**, the embedding may be performed by means of the following embedding function:

$$Emb_U(x_j) = \frac{1}{|x_j|} \sum_{i \in x_y} \theta_i^{Emb_U}$$

where $U \in \{V, \textbf{D}\}$, $\theta_i^{Emb_U}$ is the embedding of element $i$, $x_j$ is the corresponding element (that is, $v_j$ or $d_j$), $i = |x_j|$. The cardinality for $d_j$ is defined by the number of words in the related documents. The cardinality for $v_j$ is always 1; it is an indicator for the entity in the graph.

Second, as shown at 210 for the nodes $V$ and at 212 for the documents $\boldsymbol{D}$:negative sampling may be applied to randomly select $V_p^-$ and $\boldsymbol{D}_p^-$, where $V_s^- \subseteq V_p^-$ and $\boldsymbol{D}_s^- \subseteq \boldsymbol{D}^-$. That is, these sets contain incorrect tails for the selected head and relation. Negative sampling may be performed in accordance with the approach described in Garcia-Duran, Alberto, and Mathias Niepert: "KBlrn: End-to-end learning of knowledge base representations with latent, relational, and numerical features", in Proceedings of the 34th Conference on Uncertainty in Artificial Intelligence (2018).

Analogously to the embedding of the positive elements, the set of negative elements may be embedded as follows, as shown at 214 for the nodes Vand at 216 for the documents $\boldsymbol{D}$:

$$Emb_U^{NEG}(Y_s^-) = \frac{1}{|Y_s^-|} \sum_{y_s^- \in Y_s^-} Emb_U(y_s^-)$$

where $Y_s^-$ is the set of negative samples (that is, $V_s^-$ or $\boldsymbol{D}_s^-$). The cardinalities of both $V_s^-$ and $\boldsymbol{D}_s^-$ are equal to the number of negative samples. The number of negative samples is a hyperparameter of the method.

The embedding of the relation, as shown at 218, is defined as follows:

$$Emb^R(r_k) = \theta_k^{Emb^R}$$

where k is the index of a particular relationship type. (According to the exemplary embodiment described herein, there are six relationship types.)

More concretely, $Emb_V$ is a neural network which gives an embedding for each entity in the KG, while $Emb^R$ is a neural network which gives an embedding for each relationship type. Additionally, $Emb_D$ is a neural network which gives an embedding for each token in the vocabulary, and the embedding for a document is simply the average embedding of all of its tokens. $Emb_U^{NEG}$ is a simple function that gives the average embedding of all entities passed to it.

### *Learning unified embeddings*

According to embodiments of the present invention, the neural network system 200 is configured to calculate the Euclidean distance between the embedding of the positive sample $x_j$ and the center of the negative samples $Y_s^-$:

$$distance_U(x_j, Y_s^-) = \left\| Emb_U(x_j) - Emb_U^{NEG}(Y_s^-) \right\|^2$$

as shown at 220 for the nodes $V$ and at 222 for the documents $\boldsymbol{D}.$

Further, as shown at 224, the distance between $distance_V$ and $distance_D$ is calculated as $L_{offset}$:

$$L_{offset} = \left\| distance_V(v_j, V_s^-) - distance_D(d_j, \boldsymbol{D}_s^-) \right\|$$

That is, $distance_V(v_j, V_s^-)$ gives the distance between the embedding from the KG for the positive sample and the center of the negative ones, while $distance_D(d_j, \boldsymbol{D}_s^-)$ gives the distance between the embedding from the document for the positive sample and the center of the negative ones.

It is important to note that in standard approaches, these distances may differ greatly; in such cases, $L_{offset}$ will be large. In contrast, in accordance with embodiments of the present invention, the neural network system 200 aims at minimizing the $L_{offset}$. Hence, the embedding distance between $v_j$ and $d_j$ to the respective negative embedded samples $V_s^-$ and $\boldsymbol{D}_s^-$ will be minimized. As a result, the model explicitly accounts for similarity in the KG when learning embeddings for the documents and vice versa. That is, the model explicitly unifies the embeddings of the KG structure and the associated documents, which distinguishes the solution according to the present invention from other known prior art methods.

It is important to note that in accordance with the above implementation, the model adjusts the learned embeddings of the entities to find a balance between the evidence in the KG and the documents. Fig. 1, Part 3 illustrates an example how this can affect the embedding space. The left side of the figure shows the embeddings learned by standard approaches: Gene 1017 is moderately close in the embedding space to the other Asthma-associated proteins due to the similar documents. However, as can be seen in Fig. 1, Part 2, Gene 1017 is not close to the others in the KG, so it is not clear if it should be associated with Asthma. On the right side of the figure of Part 3, though, one can see the impact of using $L_{offset}$: Due to the unified learning as explained above, the document embeddings inform the KG embeddings. Consequently, it is now clear that also Gene 1017 should be associated with Asthma.

### *Learning accurate relation predictions*

According to embodiments of the invention, in addition to $L_{offset}$, the neural network component 200 may also keep track of the prediction accuracy of the model, that is, it measures whether learned weights of the embedding functions are reliable for accurately predicting relations. In particular, the neural network component 200 may use the learned weights of the embedding layers (*Emb$_V$*, *Emb$_D$*, and *Emb$^R$*) to predict the relationships known to be in the KG 100.

More specifically, as already mentioned above, embodiments of the invention aim at predicting the true tail of a triple when the head and relationship type are fixed. That is, assuming, for example a particular disease as a given tail entity *h* (such as "Cardiomyopathy" in accordance with the example given above) and a particular relation type $r_k$ ("associated_with"), the aim is to predict a correct tail entity *t* for the triple ($h, r, t$). Hereinafter, the node and document associated with the head are referred to as $v_{head}$ and $d_{read}$, respectively. The prediction result is calculated by a function denoted $L_{predict}$.

In contrast to $L_{offset}$, $L_{predict}$ does not average the embeddings of the negative samples (from $V_s^-$ and $\boldsymbol{D}_s^-$), but rather compares all of them to the correct tail ($v_j$ and $d_j$) to measure the predictive performance. In particular, according to an embodiment, the embeddings of the fixed head and the fixed relationship type may be combined (multiplied element-wise, "•") to group the nodes (as shown at 226) as well as the documents (as shown at 228) in the embedding space by the relation type:

$$Group_U(x_{head}, r_k) = Emb_U(x_{head}) \bullet Emb^R(r_k)$$

Finally, according to embodiments, the score of each candidate tail (that is, *j* as well as each negative sample) is calculated as the dot product of its embedding with $Group_U(x_{head}, r_k)$ as follows (as shown at 230 for the nodes and at 232 for the documents):

$$Predict_U(x_{head}, r_k, x_j) = Group_U(x_{head}, r_k) \cdot Emb_U(x_j)$$

Each score is a measure of how likely the respective entity correctly completes the triple ($h, r, ?$). In the optimal case, the score of the negative samples should be 0 while the score of a true tail should be 1.

As shown in Fig. 2, the above step is performed separately for both modalities, i.e. nodes and documents, and the resulting vectors are combined (added element-wise) into a score as follows (shown at 234):

$$Predict(head, r_k, j) = Predict_V(v_{head}, r_k, v_j) + Predict_D(d_{head}, r_k, d_j)$$

This score is computed for all negative samples and the true tail. Since the exemplary embodiment discussed herein, considers two modalities, the combined score for the true tail should be 2, while the combined score for the negative samples should still be 0. In more general terms, i.e. in case of considering *n* different modalities, the above equation for *Predict(head, $r_k$, j)* would be composed of *n* summands and the combined score for the true tail would be *n*. The calculation of $L_{offset}$ is exponential in the number of included modalities.

In the exemplary embodiment of Fig. 2 with two modalities, the resulting scores are considered to compute a binary cross entropy loss (as shown at 236):

$$L_{predict} = BinaryCrossEntropy\left(\begin{vmatrix} Predict(head, r_k, j) \\ Predict(head, r_k, y_1^-) \\ \vdots \\ Predict(head, r_k, y_m^-) \end{vmatrix}, \begin{vmatrix} 2 \\ 0 \\ \vdots \\ 0 \end{vmatrix}\right),$$

where $y_i^-$ is the *i$^{th}$* negative sample.

Finally, according to embodiments of the present invention, both loss values may be combined to measure the performance of the respective machine learning model, for example as follows:

$$L = \propto L_{predict} + (1-\propto)L_{offset},$$

where $\propto \in [0,1]$ is a hyperparameter, which controls the tradeoff between accuracy and the unified embeddings. Standard backpropagation algorithms may then be used to update the parameters of the $Emb_V$, $Emb^R$ and $Emb_D$ so that the predictions become more accurate while still respecting the unification of the KG and document embeddings; that is, the parameters are updated so that $L$ is minimized.

As an example, reference is made to Fig. 1, Part 2. By taking into account the graph structure and the biomedical documents and unifying their embeddings, the mechanism described above recognizes that also "Gene 1017" ($P_3$) is also associated with disease D1, i.e. Asthma. As a result, the present invention identified a novel relation ($e^{d\_aw\_p}$) which can be investigated in more detail and considered while developing novel drugs. As will be appreciated by those skilled in the art, this is just an example for one particular edge type and similar scenarios exist for the remaining relations that would also be revealed by the present invention.

***Ranking novel relationships***

According to embodiments of the invention, after training the model, the parameters of the embedding functions $Emb_V$, $Emb^R$ and $Emb_D$ are set such that true triples result in a high $Predict(\cdot)$ value, while incorrect triples result in low values.

That is, the trained model can answer queries of the following forms: (h, r, ?), (h, ?, t), and (?, r, t). Specifically, when given two of the elements, $Predict(\cdot)$ is calculated for all possible values of the missing element of the triple. The respective scores are used to give a ranking from the most-likely (highest-value) to least-likely (lowest) completions of the triple. For cases in which correct completions of the query are known, such rankings can be evaluated using standard information retrieval metrics such as area under the receiver operating characteristic curve, precision@k, and area under the precision-recall curve.

As already mentioned above, the pipeline outlined in Fig. 2 is not restricted to only learning embeddings of nodes and documents jointly. Indeed, according to further embodiments of the invention, the system could be easily enhanced by adding additional objects of other data modalities, such as images. In that case, the loss function $L_{offset}$ would computed for each pair; hence, considering nodes, documents, and images would result in three pairs: (nodes, documents), (nodes, images), and (documents, images). Therefore, $L_{offset}$ would be computed individually for each pair as described as described above and as shown in Fig. 2 at 224 for the pair (nodes, documents). However, it should be noted that this approach still ensures that all modalities are learned jointly. Generalizing this, an overall loss can be computed as follows:

$$L = xL_{predict} + y_1 L_{offset} \cdots + y_n L_{offset}$$

where $n = \binom{m}{2}$, $m$ represent the number of data modalities, and $x + \sum_{i=1}^{n} y_i = 1$.

In an embodiment, the present invention relates to a method for identifying disease-gene associations, which may be applied, e.g., in connection with automating CRISPR-Cas9 knockdown experiments (as described, for instance, in Zhou, Y., Zhu, S., Cai, C. et al. High-throughput screening of a CRISPR/Cas9 library for functional genomics in human cells. Nature 509, 487-491 (2014). https://doi.org/10.1038/nature13166) to understand the effect of specific genes on diseases. Briefly, CRISPR-Cas9 knockdown experiments use a guide RNA which targets a specific gene; after applying the CRISPR-Cas9 compound with a particular guide RNA, the targeted gene no longer functions. (It is "knocked down.") The goal of this system is to identify the genes most relevant for a disease by prioritizing genes for knockdown experiments. (Presumably, these genes are then good candidates for drug discovery, but we that idea is not further pursued in the present disclosure.)

In this case, nodes and edges in the knowledge graph correspond to genes, chemicals, and diseases, as already described and as shown in Fig. 1, Part 2. Further, a set of guide RNAs for known, specific genes are physically available in a storage container. Such sequence libraries are publicly available (for reference see, e.g., Doench, J., Fusi, N., Sullender, M. et al. Optimized sgRNA design to maximize activity and minimize off-target effects of CRISPR-Cas9. Nat Biotechnol 34, 184-191 (2016). https://doi.org/10.1038/nbt.3437). A robot is able to extract a specific guide RNA from storage and place it in a designated set of wells in a microtiter plate. For example, the BenchBot Robot by Agilent (for reference, see https://www.bioprocessonline.com/doc/benchbot-robot-0001) is capable of doing this.

First, a disease of interest is selected. Importantly, a cell line must be available which is similar to that disease; for example, KG-1 and HL-60 are cell lines for leukemia (for reference, see Koeffler HP, Golde DW. Human myeloid leukemia cell lines: a review. Blood, 1980 Sep;56(3):344-50, available under https://www.ncbi.nlm.nih.gov/pubmed/6996765).

Second, the robot distributes Cas9 into all of the wells of the plate. Then, a method of learning novel relationships among various biological entities according to embodiments of the present invention may be used to identify the genes mostly likely to be associated with the disease. That is, the method predicts "gene associated with disease" relationships for the selected disease and takes the top-ranked genes. The number of selected genes may be determined by the number of wells on the plate. Each gene is assigned to one or more wells on the plate. The robot automatically selects the respective guide RNAs from storage and places them in the microtiter plate in known wells in order to co-incubate with Cas9. After sufficient time, the cells from the cell line are combined into the system using an appropriate technique, such as electroporation (https://www.takarabio.com/learning-centers/stem-cell-research/technical-notes/gene-editing-in-hips-cells/generating-clonal-hips-cell-lines-deficient-in-cd81).

Finally, another assay may be used to evaluate the effect of knocking down each gene in the cell line. For example, Konstantinos Tzelepis, et al. A CRISPR Dropout Screen Identifies Genetic Vulnerabilities and Therapeutic Targets in Acute Myeloid Leukemia. Cell Reports. Volume 17, Issue 4, 18 October 2016, pages 1193-1205 use RNA sequencing to identify genetic pathways which function differently in the knocked down cells compared to the unaltered cell line.

In an alternative embodiment, the present invention relates to a method for identifying disease-gene associations, which may be applied, e.g., in connection with a personalized medicine system for developing personalized drugs. The goal of such a system is to select the best chemical to treat a disease for a particular patient. In this case, nodes in the knowledge graph (KG) again correspond to genes, chemicals, and diseases, as shown in Fig. 1, Part 2. Further, a subset of chemicals included in the KG are physically available in a storage container. A robot is able to extract a specific chemical from storage and place it in a designated set of wells in a microtiter plate. For example, the BenchBot Robot by Agilent is capable of doing this.

First, a patient is diagnosed with a particular disease. Second, a biopsy is taken from the patient; depending on the type of disease, the biopsy may be liquid (for example, for leukemias) or solid (for example, for ovarian cancer). Relevant cells are extracted from the biopsy using standard approaches. For example, a standard protocol (for reference, see Panda, S. K. and Ravindran, B. (2013). Isolation of Human PBMCs. Bio-protocol3(3): e323. DOI: 10.21769/BioProtoc.323) is available to extract immune cells from blood. The robot distributes the cells into wells on one or more microtiter plates. Then, a method of learning novel relationships among various biological entities according to embodiments of the present invention may be used to identify the chemicals most likely to treat the disease. That is, the method predicts "chemical treats disease" relationships for the patient's disease and takes the top-ranked chemicals. The number of selected chemicals may be determined by the number of wells on the plate. Each chemical is assigned to one or more wells on the plate. The robot automatically selects those chemicals from storage and places them in the microtiter plate in known wells.

Another assay is used to determine the effect of each chemical on the patient cells. As an example, high throughput ELISpot assays such as the one available from R&D Systems (for reference, see https://www.rndsystems.com/products/dual-color-elispot-kits) could be used to determine which chemicals elicit a response from immune system cells. The number of spots indicates how effective the chemical is for the patient. Finally, the most effective chemical for the particular patient's case may be selected for use in treatment.

As will be appreciated by those skilled in the art, the methods described herein are dependent on having enough information to construct the KG and documents to begin with. In some domains, this could be problematic. However, in the biomedical domain, and specifically in the field of the biomedical application scenarios described above, sufficient data is already available.

Many modifications and other embodiments of the invention set forth herein will come to mind to the one skilled in the art to which the invention pertains having the benefit of the teachings presented in the foregoing description and the associated drawings. Therefore, it is to be understood that the invention is not to be limited to the specific embodiments disclosed and that modifications and other embodiments are intended to be included within the scope of the appended claims. Although specific terms are employed herein, they are used in a generic and descriptive sense only and not for purposes of limitation.

## Claims

1. A computer-implemented method of learning novel relationships among biological entities including chemicals, genes and/or proteins, and diseases, the method comprising:

   establishing a knowledge graph (100) wherein each of the entities is represented as a node (110) and each relationship between the entities is represented as an edge (120) between the respective nodes (110);
   annotating entities in the knowledge graph (100) with objects (130) of one or more data modalities;
   training a neural network system (200) with the knowledge graph (100), wherein the neural network system (200) treats the knowledge graph (100) and the objects (130) of a respective one of the data modalities in a unified manner by jointly learning embeddings of the nodes (110) from the knowledge graph (100) and embeddings of the objects (130) of the respective one of the data modalities, wherein the neural network system (200) is configured to minimize for a particular positive sample a loss function that calculates the Euclidean distance between a distance between the embeddings of the nodes (110) from the knowledge graph (100) for the positive sample and a center of the respective negative samples and a distance between the embeddings from the object (130) of a particular of the data modalities for the positive sample and a center of the negative samples;

using the learned embeddings for identifying novel relationships among the entities including selecting a particular disease and using the neural network system (200) to predict for the selected disease relationships of the form 'gene/protein associated with disease' or of the form 'chemical treats disease';

ranking the predicted genes/proteins or chemicals according to a likelihood of the respective one of the predicted genes/proteins or chemicals to be associated with the selected disease or to treat the selected disease; and selecting a predefined number of the top-ranked genes/proteins as candidates for a knockdown experiment or of the top-ranked chemicals as candidates for personalized drug development.

2. The method according to claim 1, wherein the one or more data modalities include objects (130) in form of biomedical documents (140) describing details of the entities and/or in form of images.

3. The method according to claim 1 or 2, wherein a machine learning model maintained by the neural network system (200) is trained with a single sample at a time,

wherein a sample is provided as a triple composed of a head entity, a tail entity and a corresponding relation type between the head entity and the tail entity, and

wherein, for a particular head entity and a particular relation type, the tail entities are grouped into a positive subset and into a negative subset in such a way that for each tail entity in the positive subset it holds that a respective triple exists in the knowledge graph (100), while for each tail entity in the negative subset it holds that no respective triple exists in the knowledge graph (100).

4. The method according to any of claims 1 to 3, wherein the loss function is calculated individually for each pair of utilized data modalities.

5. The method according to any of claims 1 to 4, wherein the neural network system (200) measures a prediction accuracy of a respective one of a trained machine learning model based on learned weights of the embeddings.

6. The method according to any of claims 1 to 5, further comprising introducing a hyperparameter for controlling a tradeoff between a prediction accuracy and the unified embeddings.

7. A computer system for learning novel relationships among biological entities including chemicals, genes and/or proteins, and diseases, the computer system comprising memory and one or more processors which, alone or in combination, are configured to provide for execution of a method comprising:

establishing a knowledge graph (100) wherein each of the entities is represented as a node (110) and each relationship between the entities is represented as an edge (120) between the respective nodes (110);

annotating entities in the knowledge graph (100) with objects (130) of one or more data modalities;

training a neural network system (200) with the knowledge graph (100), wherein the neural network system (200) treats the knowledge graph (100) and the objects (130) of a respective one of the data modalities in a unified manner by jointly learning embeddings of the nodes (110) from the knowledge graph (100) and embeddings of the objects (130) of the respective one of the data modalities, wherein the neural network system (200) is configured to minimize for a particular positive sample a loss function that calculates the Euclidean distance between a distance between the embeddings of the nodes (110) from the knowledge graph (100) for the positive sample and a center of the respective negative samples and a distance between the embeddings from the object (130) of a particular of the data modalities for the positive sample and a center of the negative samples;

using the learned embeddings for identifying novel relationships among the entities including selecting a particular disease and using the neural network system (200) to predict for the selected disease relationships of the form 'gene/protein associated with disease' or of the form 'chemical treats disease';

ranking the predicted genes/proteins or chemicals according to a likelihood of the respective one of the predicted genes/proteins or chemicals to be associated with the selected disease or to treat the selected disease; and selecting a predefined number of the top-ranked genes/proteins as candidates for a knockdown experiment or of the top-ranked chemicals as candidates for personalized drug development.

8. The computer system according to claim 7, wherein the loss function is calculated individually for each pair of utilized data modalities.

9. The computer system according to claim 7 or 8, wherein the neural network system (200) measures a prediction accuracy of a respective one of a trained machine learning model based on learned weights of the embeddings.

10. The computer system according to any of claims 7 to 9, further comprising a biomedical documents mining component (150) that is configured to prepare biomedical documents based on a common vocabulary and to generate a set of biomedical documents, where each element of the set is a multiset of tokens from the vocabulary.

11. A non-transitory computer-readable medium having instructions thereon, which, upon execution by one or more processors, alone or in combination, and using memory, provides for execution of a method comprising:

establishing a knowledge graph (100) of biological entities including chemicals, genes and/or proteins, and diseases,
wherein each of the entities is represented as a node (110) and each relationship between the entities is represented as an edge (120) between the respective nodes (110);
annotating entities in the knowledge graph (100) with objects (130) of one or more data modalities;
training a neural network system (200) with the knowledge graph (100), wherein the neural network system (200) treats the knowledge graph (100) and the objects (130) of a respective one of the data modalities in a unified manner by jointly learning embeddings of the nodes (110) from the knowledge graph (100) and embeddings of the objects (130) of the respective one of the data modalities, wherein the neural network system (200) is configured to minimize for a particular positive sample a loss function that calculates the Euclidean distance between a distance between the embeddings of the nodes (110) from the knowledge graph (100) for the positive sample and a center of the respective negative samples and a distance between the embeddings from the object (130) of a particular of the data modalities for the positive sample and a center of the negative samples;
using the learned embeddings for identifying novel relationships among the entities including selecting a particular disease and using the neural network system (200) to predict for the selected disease relationships of the form 'gene/protein associated with disease' or of the form 'chemical treats disease';
ranking the predicted genes or chemicals according to a likelihood of the respective one of the predicted genes/proteins or chemicals to be associated with the selected disease or to treat the selected disease; and
selecting a predefined number of the top-ranked genes/proteins as candidates for a knockdown experiment or of the top-ranked chemicals as candidates for personalized drug development.

**Patentansprüche**

1. Computerimplementiertes Verfahren zum Lernen neuer Beziehungen zwischen biologischen Entitäten umfassend chemische Substanzen, Gene und/oder Proteine und Krankheiten, wobei das Verfahren umfasst:

Erstellen eines Wissensgraphen (100), in dem jede der Entitäten als ein Knoten (110) repräsentiert ist und jede Beziehung zwischen den Entitäten als eine Kante (120) zwischen den jeweiligen Knoten (110) repräsentiert ist;
Annotieren von Entitäten in dem Wissensgraphen (100) mit Objekten (130) einer oder mehrerer Datenmodalitäten;
Trainieren eines neuronalen Netzwerksystems (200) mit dem Wissensgraphen (100), wobei das neuronale Netzwerksystem (200) den Wissensgraphen (100) und die Objekte (130) einer jeweiligen Datenmodalität auf einheitliche Weise behandelt, indem es Einbettungen der Knoten (110) aus dem Wissensgraphen (100) und Einbettungen der Objekte (130) der jeweiligen Datenmodalität gemeinsam lernt, wobei das neuronale Netzwerksystem (200) so konfiguriert ist, dass es für eine bestimmte positive Probe eine Verlustfunktion minimiert, die den euklidischen Abstand zwischen einem Abstand zwischen den Einbettungen der Knoten (110) aus dem Wissensgraphen (100) für die positive Probe und einem Zentrum der jeweiligen negativen Proben und einem Abstand zwischen den Einbettungen aus dem Objekt (130) einer bestimmten der Datenmodalitäten für die positive Probe und einem Zentrum der negativen Proben berechnet;
Verwenden der gelernten Einbettungen zum Identifizieren neuer Beziehungen zwischen den Entitäten, einschließlich Auswählen einer bestimmten Krankheit und Verwenden des neuronalen Netzwerksystems (200), um für die ausgewählte Krankheit Beziehungen der Form "Gen/Protein assoziiert mit Krankheit" oder der Form "chemische Substanz behandelt Krankheit" vorherzusagen;

Einstufen der vorhergesagten Gene/Proteine oder chemischen Subtanzen entsprechend einer Wahrscheinlichkeit, dass das jeweilige der vorhergesagten Gene/Proteine oder chemischen Subtanzen mit der ausgewählten Krankheit assoziiert ist oder die ausgewählte Krankheit behandelt; und

Auswählen einer vordefinierten Anzahl der am höchsten eingestuften Gene/Proteine als Kandidaten für ein Knockdown-Experiment oder der am höchsten eingestuften chemischen Subtanzen als Kandidaten für eine personalisierte Arzneimittelentwicklung.

2. Verfahren nach Anspruch 1, wobei die eine oder mehreren Datenmodalitäten Objekte (130) in Form von biomedizinischen Dokumenten (140), die Details der Entitäten beschreiben, und/oder in Form von Bildern umfassen.

3. Verfahren nach Anspruch 1 oder 2, wobei ein von dem neuronalen Netzwerksystem (200) gepflegtes maschinelles Lernmodell mit jeweils einer einzelnen Probe trainiert wird,

wobei eine Probe als Triple bereitgestellt wird, die aus einer Kopfentität, einer Schwanzentität und einem entsprechenden Beziehungstyp zwischen der Kopfentität und der Schwanzentität besteht, und

wobei für eine bestimmte Kopfentität und einen bestimmten Beziehungstyp die Schwanzentitäten in eine positive Teilmenge und in eine negative Teilmenge so gruppiert werden, dass für jede Schwanzentität in der positiven Teilmenge ein entsprechendes Triple in dem Wissensgraphen (100) vorhanden ist, während für jede Schwanzentität in der negativen Teilmenge kein entsprechendes Triple in dem Wissensgraphen (100) vorhanden ist.

4. Verfahren nach einem der Ansprüche 1 bis 3, wobei die Verlustfunktion für jedes Paar verwendeter Datenmodalitäten individuell berechnet wird.

5. Verfahren nach einem der Ansprüche 1 bis 4, wobei das neuronale Netzwerksystem (200) eine Vorhersagegenauigkeit eines jeweiligen trainierten maschinellen Lernmodells auf der Grundlage von gelernten Gewichten der Einbettungen misst.

6. Verfahren nach einem der Ansprüche 1 bis 5, das ferner das Einführen eines Hyperparameters zum Steuern eines Kompromisses zwischen einer Vorhersagegenauigkeit und den vereinheitlichten Einbettungen umfasst.

7. Computersystem zum Lernen neuer Beziehungen zwischen biologischen Entitäten umfassend chemische Substanzen, Gene und/oder Proteine und Krankheiten, wobei das Computersystem einen Speicher und einen oder mehrere Prozessoren umfasst, die allein oder in Kombination so konfiguriert sind, dass sie die Ausführung eines Verfahrens ermöglichen, das Folgendes umfasst:

Erstellen eines Wissensgraphen (100), in dem jede der Entitäten als ein Knoten (110) repräsentiert ist und jede Beziehung zwischen den Entitäten als eine Kante (120) zwischen den jeweiligen Knoten (110) repräsentiert ist;

Annotieren von Entitäten in dem Wissensgraphen (100) mit Objekten (130) einer oder mehrerer Datenmodalitäten;

Trainieren eines neuronalen Netzwerksystems (200) mit dem Wissensgraphen (100), wobei das neuronale Netzwerksystem (200) den Wissensgraphen (100) und die Objekte (130) einer jeweiligen Datenmodalität auf einheitliche Weise behandelt, indem es Einbettungen der Knoten (110) aus dem Wissensgraphen (100) und Einbettungen der Objekte (130) der jeweiligen Datenmodalität gemeinsam lernt, wobei das neuronale Netzwerksystem (200) so konfiguriert ist, dass es für eine bestimmte positive Probe eine Verlustfunktion minimiert, die den euklidischen Abstand zwischen einem Abstand zwischen den Einbettungen der Knoten (110) aus dem Wissensgraphen (100) für die positive Probe und einem Zentrum der jeweiligen negativen Proben und einem Abstand zwischen den Einbettungen aus dem Objekt (130) einer bestimmten der Datenmodalitäten für die positive Probe und einem Zentrum der negativen Proben berechnet;

Verwenden der gelernten Einbettungen zum Identifizieren neuer Beziehungen zwischen den Entitäten, einschließlich Auswählen einer bestimmten Krankheit und Verwenden des neuronalen Netzwerksystems (200), um für die ausgewählte Krankheit Beziehungen der Form "Gen/Protein assoziiert mit Krankheit" oder der Form "chemische Substanz behandelt Krankheit" vorherzusagen;

Einstufen der vorhergesagten Gene/Proteine oder chemischen Substanzen entsprechend einer Wahrscheinlichkeit, dass das jeweilige der vorhergesagten Gene/Proteine oder chemischen Substanzen mit der ausgewählten Krankheit assoziiert ist oder die ausgewählte Krankheit behandelt; und

Auswählen einer vordefinierten Anzahl der am höchsten eingestuften Gene/Proteine als Kandidaten für ein Knockdown-Experiment oder der am höchsten eingestuften chemischen Substanzen als Kandidaten für eine personalisierte Arzneimittelentwicklung.

8. Computersystem nach Anspruch 7, wobei die Verlustfunktion für jedes Paar verwendeter Datenmodalitäten individuell berechnet wird.

9. Computersystem nach Anspruch 7 oder 8, wobei das neuronale Netzwerksystem (200) eine Vorhersagegenauigkeit eines jeweiligen trainierten maschinellen Lernmodells auf der Grundlage von gelernten Gewichten der Einbettungen misst.

10. Computersystem nach einem der Ansprüche 7 bis 9, das ferner eine Komponente (150) zum Durchsuchen biomedizinischer Dokumente umfasst, die so konfiguriert ist, dass sie biomedizinische Dokumente auf der Grundlage eines gemeinsamen Vokabulars aufbereitet und einen Satz biomedizinischer Dokumente erzeugt, wobei jedes Element des Satzes eine Multimenge von Token aus dem Vokabular ist.

11. Nichtflüchtiges, computerlesbares Speichermedium, das Anweisungen enthält, die bei Ausführung durch einen oder mehrere Prozessoren, allein oder in Kombination, und unter Verwendung von Speicher die Ausführung eines Verfahrens ermöglichen, das Folgendes umfasst:

Erstellen eines Wissensgraphen (100) von biologischen Entitäten umfassend chemische Substanzen, Gene und/oder Proteine und Krankheiten, wobei jede der Entitäten als ein Knoten (110) repräsentiert ist und jede Beziehung zwischen den Entitäten als eine Kante (120) zwischen den jeweiligen Knoten (110) repräsentiert ist; Annotieren von Entitäten in dem Wissensgraphen (100) mit Objekten (130) einer oder mehrerer Datenmodalitäten;

Trainieren eines neuronalen Netzwerksystems (200) mit dem Wissensgraphen (100), wobei das neuronale Netzwerksystem (200) den Wissensgraphen (100) und die Objekte (130) einer jeweiligen Datenmodalität auf einheitliche Weise behandelt, indem es Einbettungen der Knoten (110) aus dem Wissensgraphen (100) und Einbettungen der Objekte (130) der jeweiligen Datenmodalität gemeinsam lernt, wobei das neuronale Netzwerksystem (200) so konfiguriert ist, dass es für eine bestimmte positive Probe eine Verlustfunktion minimiert, die den euklidischen Abstand zwischen einem Abstand zwischen den Einbettungen der Knoten (110) aus dem Wissensgraphen (100) für die positive Probe und einem Zentrum der jeweiligen negativen Proben und einem Abstand zwischen den Einbettungen aus dem Objekt (130) einer bestimmten der Datenmodalitäten für die positive Probe und einem Zentrum der negativen Proben berechnet;

Verwenden der gelernten Einbettungen zum Identifizieren neuer Beziehungen zwischen den Entitäten, einschließlich Auswählen einer bestimmten Krankheit und Verwenden des neuronalen Netzwerksystems (200), um für die ausgewählte Krankheit Beziehungen der Form "Gen/Protein assoziiert mit Krankheit" oder der Form "chemische Substanz behandelt Krankheit" vorherzusagen;

Einstufen der vorhergesagten Gene/Proteine oder chemischen Substanzen entsprechend einer Wahrscheinlichkeit, dass das jeweilige der vorhergesagten Gene/Proteine oder chemischen Substanzen mit der ausgewählten Krankheit assoziiert ist oder die ausgewählte Krankheit behandelt; und

Auswählen einer vordefinierten Anzahl der am höchsten eingestuften Gene/Proteine als Kandidaten für ein Knockdown-Experiment oder der am höchsten eingestuften chemische Substanzen als Kandidaten für eine personalisierte Arzneimittelentwicklung.

**Revendications**

1. Procédé mis en œuvre par ordinateur d'apprentissage de nouvelles relations entre des entités biologiques incluant des produits chimiques, des gènes et/ou des protéines, et des maladies, le procédé comportant :

l'établissement d'un graphe de connaissances (100) dans lequel chacune des entités est représentée sous la forme d'un nœud (110) et chaque relation entre les entités est représentée sous la forme d'une arête (120) entre les nœuds (110) respectifs ;

l'annotation d'entités dans le graphe de connaissances (100) avec des objets (130) d'une ou plusieurs modalités de données ;

l'entraînement d'un système de réseau neuronal (200) avec le graphe de connaissances (100), dans lequel le système de réseau neuronal (200) traite le graphe de connaissances (100) et les objets (130) d'une modalité de données respective parmi les modalités de données d'une manière unifiée par apprentissage conjoint d'incorporations des nœuds (110) provenant du graphe de connaissances (100) et d'incorporations des objets (130) de la modalité de données respective parmi les modalités de données, dans lequel le système de réseau neuronal (200) est configuré pour minimiser, pour un échantillon positif particulier, une fonction de perte qui calcule la distance euclidienne entre une distance entre les incorporations des nœuds (110) du graphe de connaissances (100) pour l'échantillon positif et un centre des échantillons négatifs respectifs et une distance entre les incorporations de l'objet (130) d'une modalité de données particulière parmi les modalités de données pour l'échantillon positif et un centre des échantillons négatifs ;

l'utilisation des incorporations apprises pour identifier de nouvelles relations entre les entités incluant la sélection d'une maladie particulière et l'utilisation du système de réseau neuronal (200) pour prédire, pour la maladie sélectionnée, des relations de la forme « gène/protéine associé(e) à la maladie » ou de la forme « produit chimique traitant la maladie » ;

le classement des gènes/protéines ou produits chimiques prédits selon une probabilité que les gènes/protéines ou produits chimiques prédits respectifs parmi les gènes/protéines ou produits chimiques prédits soient associés à la maladie sélectionnée ou traitent la maladie sélectionnée ; et

la sélection d'un nombre prédéfini des gènes/protéines les mieux classés comme candidats pour une expérience d'inactivation ou des produits chimiques les mieux classés comme candidats pour le développement de médicaments personnalisés.

**2.** Procédé selon la revendication 1, dans lequel les une ou plusieurs modalités de données incluent des objets (130) sous forme de documents biomédicaux (140) décrivant des détails des entités et/ou sous forme d'images.

**3.** Procédé selon la revendication 1 ou 2, dans lequel un modèle d'apprentissage automatique géré par le système de réseau neuronal (200) est entraîné avec un seul échantillon à la fois,

dans lequel un échantillon est fourni sous la forme d'un triple composé d'une entité de tête, d'une entité de queue et d'un type de relation correspondant entre l'entité de tête et l'entité de queue, et

dans lequel, pour une entité de tête particulière et un type de relation particulier, les entités de queue sont regroupées dans un sous-ensemble positif et dans un sous-ensemble négatif de manière à ce que, pour chaque entité de queue dans le sous-ensemble positif, il soit considéré qu'un triple respectif existe dans le graphe de connaissances (100), tandis que pour chaque entité de queue dans le sous-ensemble négatif, il soit considéré qu'il n'existe aucun triple respectif dans le graphe de connaissances (100).

**4.** Procédé selon l'une quelconque des revendications 1 à 3, dans lequel la fonction de perte est calculée individuellement pour chaque paire de modalités de données utilisées.

**5.** Procédé selon l'une quelconque des revendications 1 à 4, dans lequel le système de réseau neuronal (200) mesure une précision de prédiction d'un modèle d'apprentissage automatique entraîné respectif sur la base de poids appris des incorporations.

**6.** Procédé selon l'une quelconque des revendications 1 à 5, comportant en outre l'introduction d'un hyperparamètre pour commander un compromis entre une précision de prédiction et les incorporations unifiées.

**7.** Système informatique d'apprentissage de nouvelles relations entre des entités biologiques incluant des produits chimiques, des gènes et/ou des protéines, et des maladies, le système informatique comportant une mémoire et un ou plusieurs processeurs qui, seuls ou en combinaison, sont configurés pour permettre l'exécution d'un procédé comportant :

l'établissement d'un graphe de connaissances (100) dans lequel chacune des entités est représentée sous la forme d'un nœud (110) et chaque relation entre les entités est représentée sous la forme d'une arête (120) entre les nœuds (110) respectifs ;

l'annotation d'entités dans le graphe de connaissances (100) avec des objets (130) d'une ou plusieurs modalités de données ;

l'entraînement d'un système de réseau neuronal (200) avec le graphe de connaissances (100), dans lequel le système de réseau neuronal (200) traite le graphe de connaissances (100) et les objets (130) d'une modalité de données respective parmi les modalités de données d'une manière unifiée par apprentissage conjoint d'incorporations des nœuds (110) provenant du graphe de connaissances (100) et d'incorporations des objets (130) de la modalité de données respective parmi les modalités de données, dans lequel le système de réseau neuronal (200) est configuré pour minimiser, pour un échantillon positif particulier, une fonction de perte qui calcule la distance euclidienne entre une distance entre les incorporations des nœuds (110) du graphe de connaissances (100) pour l'échantillon positif et un centre des échantillons négatifs respectifs et une distance entre les incorporations de l'objet (130) d'une modalité de données particulière parmi les modalités de données pour l'échantillon positif et un centre des échantillons négatifs ;

l'utilisation des incorporations apprises pour identifier de nouvelles relations entre les entités incluant la sélection d'une maladie particulière et l'utilisation du système de réseau neuronal (200) pour prédire, pour la maladie sélectionnée, des relations de la forme « gène/protéine associé(e) à la maladie » ou de la forme « produit chimique traitant la maladie » ;

le classement des gènes/protéines ou produits chimiques prédits selon une probabilité que les gènes/protéines ou produits chimiques prédits respectifs parmi les gènes/protéines ou produits chimiques prédits soient associés à la maladie sélectionnée ou traitent la maladie sélectionnée ; et

la sélection d'un nombre prédéfini des gènes/protéines les mieux classés comme candidats pour une expérience d'inactivation ou des produits chimiques les mieux classés comme candidats pour le développement de médicaments personnalisés.

8. Système informatique selon la revendication 7, dans lequel la fonction de perte est calculée individuellement pour chaque paire de modalités de données utilisées.

9. Système informatique selon la revendication 7 ou 8, dans lequel le système de réseau neuronal (200) mesure une précision de prédiction d'un modèle d'apprentissage automatique entraîné respectif sur la base de poids appris des incorporations.

10. Système informatique selon l'une quelconque des revendications 7 à 9, comportant en outre un composant d'exploration de documents biomédicaux (150) qui est configuré pour préparer des documents biomédicaux sur la base d'un vocabulaire commun et pour générer un ensemble de documents biomédicaux, chaque élément de l'ensemble étant un multiensemble de jetons du vocabulaire.

11. Support non transitoire lisible par ordinateur ayant des instructions sur celui-ci, qui, lors de leur exécution par un ou plusieurs processeurs, seules ou en combinaison, et à l'aide d'une mémoire, permettent l'exécution d'un procédé comportant :

l'établissement d'un graphe de connaissances (100) d'entités biologiques incluant des produits chimiques, des gènes et/ou des protéines, et des maladies,

dans lequel chacune des entités est représentée sous la forme d'un nœud (110) et chaque relation entre les entités est représentée sous la forme d'une arête (120) entre les nœuds (110) respectifs ;

l'annotation d'entités dans le graphe de connaissances (100) avec des objets (130) d'une ou plusieurs modalités de données ;

l'entraînement d'un système de réseau neuronal (200) avec le graphe de connaissances (100), dans lequel le système de réseau neuronal (200) traite le graphe de connaissances (100) et les objets (130) d'une modalité de données respective parmi les modalités de données d'une manière unifiée par apprentissage conjoint d'incorporations des nœuds (110) provenant du graphe de connaissances (100) et d'incorporations des objets (130) de la modalité de données respective parmi les modalités de données, dans lequel le système de réseau neuronal (200) est configuré pour minimiser, pour un échantillon positif particulier, une fonction de perte qui calcule la distance euclidienne entre une distance entre les incorporations des nœuds (110) du graphe de connaissances (100) pour l'échantillon positif et un centre des échantillons négatifs respectifs et une distance entre les incorporations de l'objet (130) d'une modalité de données particulière parmi les modalités de données pour l'échantillon positif et un centre des échantillons négatifs ;

l'utilisation des incorporations apprises pour identifier de nouvelles relations entre les entités incluant la sélection d'une maladie particulière et l'utilisation du système de réseau neuronal (200) pour prédire, pour la maladie sélectionnée, des relations de la forme « gène/protéine associé(e) à la maladie » ou de la forme « produit chimique traitant la maladie » ;

le classement des gènes ou produits chimiques prédits selon une probabilité que les gènes/protéines ou produits chimiques prédits respectifs parmi les gènes/protéines ou produits chimiques prédits soient associés à la maladie sélectionnée ou traitent la maladie sélectionnée ; et

la sélection d'un nombre prédéfini des gènes/protéines les mieux classés comme candidats pour une expérience d'inactivation ou des produits chimiques les mieux classés comme candidats pour le développement de médicaments personnalisés.

Fig. 1

Fig. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2019370616 A1

**Non-patent literature cited in the description**

- **GARCIA-DURAN** ; **ALBERTO** ; **MATHIAS NIE-PERT**. KBlrn: End-to-end learning of knowledge base representations with latent, relational, and numerical features. *Proceedings of the 34th Conference on Uncertainty in Artificial Intelligence*, 2018
- **ZHOU, Y.** ; **ZHU, S** ; **CAI, C. et al.** High-throughput screening of a CRISPR/Cas9 library for functional genomics in human cells.. *Nature*, 2014, vol. 509, 487-491
- **DOENCH, J.** ; **FUSI, N.** ; **SULLENDER, M. et al.** Optimized sgRNA design to maximize activity and minimize off-target effects of CRISPR-Cas9. *Nat Biotechnol*, 2016, vol. 34, 184-191, https://doi.org/10.1038/nbt.3437
- **KOEFFLER HP** ; **GOLDE DW**. Human myeloid leukemia cell lines: a review.. *Blood,*, September 1980, vol. 56 (3), 344-50, https://www.ncbi.nlm.nih.gov/pubmed/6996765
- **KONSTANTINOS TZELEPIS et al.** A CRISPR Dropout Screen Identifies Genetic Vulnerabilities and Therapeutic Targets in Acute Myeloid Leukemia. *Cell Reports.*, 18 October 2016, vol. 17 (4), 1193-1205
- **PANDA, S. K.** ; **RAVINDRAN, B.** Isolation of Human PBMCs. *Bio-protocol*, 2013, vol. 3 (3), e323